# EUROPEAN PATENT APPLICATION

(11) **EP 1 391 449 A1**
(43) Date of publication of application: **25.02.2004**
(21) Application number: 03018116.8
(22) Date of filing: 08.08.2003
(51) Int. Cl.: C07C 227/18, C07C 229/24

(54) **Hydrolysis of amino acid diesters**

(30) Priority: 09.08.2002 US 215728
(71) Applicant: PCBU Services, Inc., Wilmington, Delaware 19801 (US)
(72) Inventor: Behrendt, L. Kirk, Glendale Heights, Illinois 60139 (US)
(74) Representative: Klunker . Schmitt-Nilson . Hirsch

(57) **Abstract**

The present invention provides a method for preparing an amino acid monoester from an amino acid diester without the use of an enzyme. Particularly, the method involves hydrolysis of an amino acid diester in the presence of a non-enzyme additive and under reaction conditions that affect selective hydrolysis of the α-ester group. The reaction conditions comprise a specific pH, temperature, and vapor pressure.

## Description

### FIELD OF INVENTION

The present invention relates to a method for preparing monoesters from diesters. Particularly, the present invention relates to selective hydrolysis of an α-ester group of an amino acid diester.

### BACKGROUND OF THE INVENTION

It is known that amino acid esters may be prepared by a number of methods. For example, acidic amino acids or derivatives thereof may be subjected to esterification or transesterification with an alcohol in the presence of a suitable catalyst. However, a pure product is difficult to achieve by such methods.

Amino acid monoesters have been prepared by the regioselective hydrolysis of amino acid diesters. A regioselective reaction is one in which one direction of bond making or breaking occurs preferentially over all other possible directions. Regioselective hydrolysis of amino acid diesters is usually facilitated by an enzyme. Pig Liver Esterase (PLE) has been found to be effective in hydrolysis of an α-ester group of N-protected or unprotected aspartic acid and glutamic acid (U.S. Patent Nos. 5,773,261 and 5,928909). The PLE-facilitated enzymatic hydrolysis method has been used in the preparation of aspartic acid-β monoester and glutamic acid-γ-monoester.

In addition to PLE, other enzymes have been shown to be functional in regioselective hydrolysis of certain amino acid esters. For example, Porcine Pancreatic Lipase (PPL) is effective in regioselective hydrolysis of dialkyl amino acid esters. However, the effect of PPL can be observed only when the substrate is N-protected.

A disadvantage of enzymatic hydrolysis methods is that they are enzyme and/or substrate dependent. The cost and the availability of the enzyme or the substrate may be limiting. Moreover, product inhibition may occur.

A method for non-enzymatic selective hydrolysis has been disclosed for dimethyl L-aspartate hydrochloride (Gmeiner, P, et al., J. Org. Chem. 1990, 55, 3068-3074). According to this particular method, cupric carbonate basic (CuCO₃ Cu(OH)₂) and dimethyl L-aspartate hydrochloride are stirred in a mixture of ethanol and water. The mixture is heated at 70°C for 2 hours, after which the mixture is cooled to room temperature. Then, excess hydrogen sulfide (H₂S) is passed through the reaction mixture. The mixture is then filtered. The resulting filtrate is evaporated to leave a product containing L-aspartic acid β-methyl ester. One disadvantage of this method is that it requires the use of a heavy metal complex, which is not environmentally friendly. Another disadvantage of this method is that it requires the use of hydrogen sulfide, which is known as a toxic gas.

### SUMMARY OF THE INVENTION

The present invention provides a method for preparing an amino acid monoester from an amino acid diester without using an enzyme. The method involves non-enzymatic hydrolysis of an amino acid diester in the presence of at least one additive under specific reaction conditions. The amino acid diester used as the substrate may include different diesters of aspartic acid, glutamic acid, or any unnatural amino acid that has a side chain containing from 1 to 6 carbons. The additive may comprise at least one of a salt or compound additive, an acid additive, a base additive, and a combination thereof. The specific reaction conditions comprise suitable pH, temperature, and vapor pressure.

### DESCRIPTION OF THE FIGURES

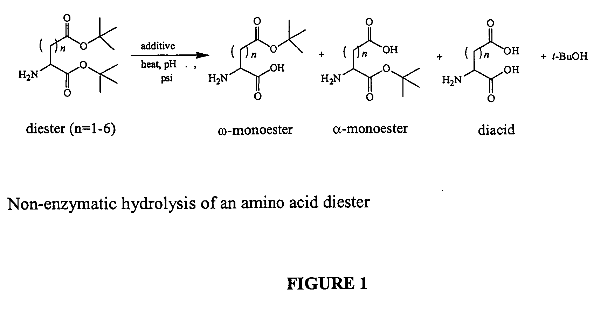

FIGURE 1: Chemical reactions showing: Hydrolysis of an amino acid diester.

### DETAILED DESCRIPTION OF THE INVENTION

For the purposes of promoting an understanding of the principles of the invention, specific language will be used to describe the embodiments of the invention. It will nevertheless be understood that no limitation of the scope of the invention is thereby intended. The invention includes any alterations and further modifications in the described products and methods and further applications of the principles of the invention which would normally occur to one skilled in the art to which the invention relates.

The present invention provides a novel method for preparing amino acid monoesters from amino acid diesters. The amino acid diesters may include natural or unnatural amino acids containing a terminal (∀) ester group and a side-chain (ω) ester groups Particularly, the method involves hydrolysis of an amino acid diester in the presence of at least one additive and under reaction conditions supporting selective hydrolysis of the α-ester group. The reaction conditions comprise a pH of between 3 and 6.5. The resulting product predominately includes an amino acid ω-monoester.

The method of the present invention is particularly suitable for hydrolysis of diesters of aspartic acid, glutamic acid, and derivatives thereof. The ω-ester group of aspartic acid is referred to as a β-ester group, and that of glutamic acid is referred to as a γ-ester group.

The method of the present invention is also suitable for hydrolysis of diesters of synthetic amino acid diester of the structure:

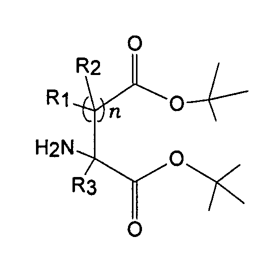

wherein n = 1-6 carbons. Each carbon may include at least one of R₁ and R₂. In addition, the α-carbon, which is the carbon atom linked to the amino group may also contain an R3. R1 and R2 can each be hydrogen, or alkyl, wherein the alkyl group is either methyl, ethyl, propyl, butyl, pentyl, or aryl group. The aryl group may include phenyl (-C₆H₅), benzyl (-CH₂C₆H₅), 1-naphthyl (-C₁₀H₇), 2-naphthyl (-C₁₀H₇). Alternatively, the aryl group may contain one or more heteroatom, such as pyridyl (C₆H₅N), furyl (C₄H₄O), thienyl (C₄H₄S), imidazoyl (C₃H₃N₂) or pyrimidyl (C₄H₄N₂). Other substitutions for R₁ and R2 may be an acyl (-COR) group, wherein R of the -COR comprises one of CH₃, C₂H₅, C₃H₇, C₄H₉, CH₂C₆H₅ and C₆H₅. In addition, halogens such as F, Cl, Br, or I may also be possible substituents for R1 and R2.

Commonly used R3 are hydrogen or alkyl groups such as methyl, ethyl, propyl or butyl.

Further, the amino acid diesters that are used as the hydrolysis substrate may be either N-protected or N-unprotected. They may contain the same or different ester groups. Examples of the ester group include a *tert*-butyl group, and an alkyl ester group.

Suitable substrates include aspartic acid-di-*tert* butyl ester, and glutamic acid-di-*tert*-butyl ester. Either an L- or a D-enantiomer of the amino acid may be used. In addition, the substrate may be in the form of a salt, or a free base. For example, the salt may include glutamic acid-di-*tert*-butyl hydrochloride salt, or aspartic acid-di-*tert*-butyl hydrochloride salt. The free base may include glutamic acid-di-*tert*-butyl ester, or aspartic acid-di-*tert*-butyl ester.

According to the present invention, at least one additive is included in the reaction mixture. Without the additive, the amino acid diester will undergo a non-selective hydrolysis, wherein either the α-ester group or the ω-ester group, or both may be hydrolyzed. It has been shown that without the additive it is likely that both the α-ester and the ω-ester groups are hydrolyzed. Thus, the predominant product may be a diacid. When at least one additive is used according to the present invention, the hydrolysis of the α-ester group is favored. As a result, the amino acid diester is predominantly converted to amino acid ω-monoester.

The additive may be any compound that functions as a buffer to keep the pH of the solution at a range of between about 3 and about 6.5. The additive may include a salt additive, which may include an alkali metal salt. Some examples of the alkali metal salt include sodium acetate, sodium citrate, sodium formate, sodium succinate, sodium carbonate, sodium bicarbonate, sodium phosphate, sodium biphoshphate and potassium phosphate. However, sodium acetate is normally used particularly due to its relatively low cost.

It is possible that the additive that is a salt additive may be made from neutralizing an acid additive with a base additive. The additive may also include at least one acid additive or at least one base additive or a combination thereof that functions as a buffer at a pH of about 3 to about 6.5. The acid additive that may be used includes any suitable organic or inorganic acid, such as acetic acid, citric acid, formic acid, succinic acid, carbonic acid and phosphoric acid. The base additive that may be used includes any suitable inorganic or organic bases. The inorganic bases that are suitable may include any alkali metal hydroxide such as sodium hydroxide, potassium hydroxide, calcium hydroxide, and ammonium hydroxide. Some examples of suitable organic bases include triethylamine, trimethyl amine, and Tris (hydroxymethyl) aminomethane.

The amount of the additive present in the reaction mixture may vary. Based on the amount of the substrate, about 0.5 to about 2.5 molar equivalents of the salt additive may be effective in facilitating selective hydrolysis of the α-ester group. However, 2 molar equivalents is commonly used.

The method of the present invention further comprises specific reaction conditions that support selective hydrolysis of the α-ester group. Specifically, the reaction conditions increase the acid lability of the α-ester group and improve the relative stability of ω-monoester.

In addition to the pH of about 3 to about 6.5, the reaction conditions further comprise a suitable temperature and suitable vapor pressure. The suitable temperature may range from at least about 25°C to about 60°C. However, the temperature of about 50°C has been found to be effective in most cases.

Further, the suitable vapor pressure may be at least about 0.276 Bar (4 *psi*). However, a vapor pressure of between about 0.276 Bar (4 *psi*) and about 1.724 Bar (25 *psi*) has been found to be effective in most cases.

When aspartic acid-di-*tert*-butyl ester is used as the substrate, the method of the present invention yields a product containing mainly aspartic acid-β-*tert*-butyl ester, as a result of selective hydrolysis of the α-*tert*-butyl ester group. The percent conversion may be calculated based on the amount of aspartic acid-β-*tert*-butyl ester in relation to the amount of the aspartic acid-di-*tert*-butyl ester used as the substrate. Similarly, when the glutamic acid-di-*tert*-butyl ester is used as the substrate, the method of the present invention yields a product containing mainly glutamic acid-γ-*tert*-butyl ester, as a result of selective hydrolysis of the α-*tert*-butyl ester group. The percent conversion may be calculated based on the amount of glutamic acid-γ-*tert*-butyl ester in relation to the amount of the glutamic acid-di-*tert*-butyl ester used as the substrate.

The reaction mixture fluid may contain a residual amount of amino acid diester and a relatively small amount of α-monoester and diacid. The ω-monoester may be recovered, and separated from the rest of the compounds, which may be recycled and reused.

The ω-monoesters such as aspartic acid- β-*tert*-butyl ester and glutamic acid-γ-*tert*-butyl ester may be further modified by adding an N-protecting group such as 9-fluorenylmethoxycarbonyl (FMOC) to the amino group. The method for adding the N-protecting group is known in the art. The resulting product from this particular process may include Fmoc-aspartic acid-β-*tert*-butyl ester or Fmoc-glutamic acid-γ-*tert*-butyl ester.

The method of the present invention may be generally performed in the following manner.

### Method for Initiating Hydrolysis

A suitable amount of a non-enzyme additive and water may be charged into an appropriate vessel. If the non-enzyme additive is a solid salt, the salt-water mixture may be stirred until the solid salt goes into complete solution. The pH of the solution may be adjusted to about 3.0 to about 6.5 using concentrated HCl or other acids. Then, a suitable amount of amino acid diester, as a substrate, is added to the additive solution. The order in which each component is added into the vessel is not critical. After the addition of the substrate, the pH of the solution may increase. Therefore, if necessary, additional HCl may be added to the reaction mixture in order to maintain the pH at between about 4.8 and about 5.0. The reaction mixture is heated to a suitable temperature, about 50°C. The vapor pressure within the reaction vessel is kept at about 0.276 Bar (4 *psi*) to about 1.724 Bar (25 *psi*). The reaction mixture may be stirred constantly for a suitable period until it forms a homogeneous mixture. The reaction time may depend on the reaction volume. About 4 to 5 hours may be suitable for smaller volumes, and about 24 to 36 hrs may be suitable for relatively larger volumes.

### Analysis of Samples

Samples may be taken for an online analysis from the reaction mixture. The sampling may be initiated at about 24 hours or sooner and over 1 to 2 hours intervals. After it is determined that the formation of ω-monoester exceeds a desired concentration, the reaction may be stopped.

The initial amino acid monoester formation is detected by spotting 2 µl of each reaction fluid on a F₂₅₄ silica TLC plate, with a solvent system containing chloroform : methanol: H₂O: formic acid (64:30:4:2). Spots are then visualized by spraying with a 1% (w/v) ninhydrin in ethanol followed by oven drying at 100°C for 5 minutes.

The samples may be further analyzed by an achiral high performance liquid chromatography (HPLC) method, using appropriate standards such as L-Glu, H-Glu-α-(OtBu)-OH, L-Glu-γ-(OtBu)-OH, L-Glu-(OtBu)₂·HCl.

A standard HPLC apparatus and method may be used, such as a HPLC system comprising of two pumps, a UV detector, a column heater, and a refrigerated autosampler capable of performing derivatization programs. Also, particularly, a Zorbax SB-AQ, 250 x 4.6 mm, 5 micron column may be used at 40°C column temperature, and at 4°C autosampler temperature. The mobile phase may consist of: Pump A - 36% Acetonitrile (ACN)/ 64% triethyl ammonium phosphate (TEAP) buffer (0.5% triethyl amine (TEA) (v/v)), final pH = 2.5; Pump B - 100% CAN.

With the HPLC system described above, a 10 µL of each sample may be injected and run at 1.5 ml/min using a gradient program. UV absorption may be monitored at the wavelength of 338 nm.

The measurements obtained from the HPLC analysis determine the amounts of the different compounds in the reaction fluid. Percent conversion is particularly calculated based on the amount of ω-monoester in relation to the amount of the diester used as the substrate.

The following non-limiting examples further demonstrate features of the present invention.

### EXAMPLE 1

### Non-enzymatic hydrolysis of L-glu-di-tert-butyl ester.HCl

A reaction mixture containing 670 mM of L-glu-di-*tert*-butyl ester HCl and 2 mole equivalents of sodium acetate, pH 4.9, was stirred in a round bottomed flask fitted with a condenser and Therm-o-watch™ [I2R, Cheltenhana, PA], at 50°C. Samples were taken over time intervals and analyzed by HPLC for L-glu-α-*tert*-butyl ester (α-monoester), L-glu-γ-tert-butyl ester (γ-monoester), L-glu-di-*tert*-butyl esters (diester) and L-glutamic acid (diacid).

The results which are present in TABLE I show that under the reaction condition set forth above, approximately half of the substrate (diester) was hydrolyzed within 25 hours. The product of the hydrolytic reaction included α-monoester, γ-monoester, and diacid. However, γ-monoester was predominant, indicating that the hydrolysis was selective for the α-ester group.

**TABLE I:**

| **Non-enzymatic hydrolysis of diester to form γ-monoester** | | | | |
|---|---|---|---|---|
| | **Product** | | | |
| **Time (hrs)** | **diester** **(mg/mL)** | **γ-monoester** **(mg/mL)** | **α-monoester** **(mg/mL)** | **diacid** **(mg/mL)** |
| 0 | 193.09 | 1.58 | 0.92 | 0 |
| 18.5 | 119.04 | 49.01 | 1.87 | 2.04 |
| 20.2 | 113.39 | 53.76 | 1.95 | 2.44 |
| 22.7 | 109.55 | 57.48 | 1.93 | 2.75 |
| 25.0 | 100.12 | 63.08 | 1.97 | 3.41 |
| % conversion | mM | mM | mM | mM |
| 77.6 | 384.48 | 310.37 | 9.69 | 23.17 |

### EXAMPLE 2

### Non-enzymatic hydrolysis of L-Flu-di-tert-butyl ester free base

Two reaction mixtures were prepared by adding 2.0 mL of 200 g/L of L-glu-di-*tert*-butyl ester free base as substrate, to a reaction vessel. Then 2.10 g of solid sodium acetate (NaOAc·3H₂O) was added with about 5.0 mL of water. The pH of the first reaction mixture was adjusted to a pH of about 4.7 to about 5.0 using concentrated hydrochloric acid (HCl). The pH of the second reaction mixture was adjusted to a pH of about 4.8 with concentrated sulfuric acid (H₂SO₄). The volume of each mixture was adjusted to 10 mL. At about 20 to 24 hours, the samples of the reaction mixture were analyzed. The results shown in TABLE II indicate that L-glu-di-*tert*-butyl ester free base could be used as the substrate for the non-enzyme hydrolysis method. Although at 20 to 24 hours, the conversion of the diester was not complete, the conversion resulted mainly in γ-monoester. The percent conversion to γ-monoester reached 42. 32% for the first reaction mixture and 42.55% for the second mixture.

**TABLE II:**

| **Conversion of diester using L-glu-di-*tert*-butyl ester free base as substrate** | | | | | |
|---|---|---|---|---|---|
| **Reaction Mixture** | **Product** | | | | |
| | **diester** **mM** | **γ-monoester** **mM** | **α-monoester** **mM** | **diacid** **mM** | **% conversion to γ-monoester** |
| (1) | 368.62 | 321.64 | 8.27 | 26.64 | 42.32 |
| (2) | 340 | 310.62 | 8.91 | 25.96 | 42.55 |

### EXAMPLE 3

### Non-enzymatic hydrolysis in the presence or absence of an additive

Eight reaction mixtures were prepared with different amounts of L-glu-di-*tert*-butyl ester free base (diester) as substrate. The mixtures were divided into two groups. Different amounts of sodium acetate were added into the mixtures in the first group. No sodium acetate was added to the mixtures in the second group. The reactions were run at 40°C for about 46 hours. The results in TABLE III show generally that in the absence of sodium acetate, the conversion of the diester was complete in 46 hours. However, most of the conversion resulted in the diacid. The percent conversion of the diester into γ-monoester was relatively low, but slightly increased with the decreasing concentrations of the substrate. The data in TABLE III also show that, in the presence of sodium acetate, the conversion of the diester was almost complete at 46 hours. Only relatively small amounts of the diester remained. The conversion resulted mainly in the production of γ-monoester indicating selective hydrolysis. The increased amounts of the substrate and sodium acetate had little effect on the percent conversion of the diester to γ-mono ester.

**TABLE III:**

| **The effect of sodium acetate on the conversion of the diester** | | | | | | |
|---|---|---|---|---|---|---|
| **Reaction** | | | **Product** | | | |
| **diester** | **sodium acetate** **mM** | **diester** **(mM)** | **γ-monoester** **(mM)** | **α-monoester** **(mM)** | **diacid** **(mM)** | **% conversion to γ-monoester** |
| 30g/L | 100 | 4.57 | 78.38 | 0.64 | 15.70 | 78.38 |
| 30 g/L | None | 0 | 13.73 | 1.77 | 75.99 | 13.73 |
| 40 g/L | 135 | 5.99 | 105.60 | 0.69 | 21.21 | 78.21 |
| 40 g/L | None | 0 | 12.64 | 4.33 | 152.72 | 9.37 |
| 50 g/L | 170 | 8.64 | 128.52 | 1.13 | 24.47 | 75.60 |
| 50 g/L | None | 0 | 9.59 | 5.22 | 192.07 | 5.64 |
| 60g/L | 200 | 11.21 | 158.38 | 1.52 | 31.40 | 79.19 |
| 60 g/L | None | 0 | 10.77 | 4.13 | 171.34 | 5.39 |

### EXAMPLE 4

### Non-enzymatic hydrolysis using different additives

Four reaction mixtures were each prepared by adding 100 mg/L of L-glu-di-*tert*-butyl ester (diester) as substrate, to 340 mM of one of four different buffers. The substrate was first prepared by mixing 2.0 g of the diester with 10 ml of water. To each vial, 2 mL of the diester solution was added, followed by 1.36 mL of 1M buffer and 640 µL of water. The four different buffers were sodium citrate, sodium succinate, sodium formate and sodium acetate. Sodium citrate was prepared by titration of citric acid and sodium hydroxide to a pH 5.5. Sodium succinate was prepared by titration of succinic acid and sodium hydroxide to a pH 5.5. Sodium formate was prepared by titration of formic acid with sodium hydroxide to a pH of 5.5. The reaction was run for 24 hours. The results in TABLE IV indicate that all the non-enzyme additives tested had similar effects on the percent conversion of the diester γ-monoester. The conversion to α-monoester was minimal when the reaction was run in the presence of sodium citrate and sodium formate. On the other hand, in the presence of sodium succinate or sodium acetate, the conversion to α-monoester was relatively high in this particular experiment. Nevertheless, selective hydrolysis of the α-ester group was evidenced by the % conversion to γ-monoester. After 24 hours, the percent conversion ranged from 59.91% to 65.70%.

**TABLE IV:**

| **The effect of different additives on the conversion of the diester.** | | | | | |
|---|---|---|---|---|---|
| **Reaction** | | **Product** | | | |
| | **diester** **(mM)** | **γ-monoester** **(mM)** | **α-monoester** **(mM)** | **diacid** **(mM)** | **% conversion to γ-monoester** |
| Sodium Citrate | 135.18 | 203.70 | 5.41 | 14.95 | 59.91 |
| Sodium Succinate | 4.61 | 210.10 | 177.10 | 15.63 | 61.79 |
| Sodium Formate | 121.74 | 223.38 | 6.89 | 23.11 | 65.70 |
| Sodium Acetate | 4.99 | 219.94 | 153.51 | 16.99 | 64.69 |

### EXAMPLE 5

### Non-enzymatic hydrolysis using different concentration of additive

Three reaction mixtures were prepared with 340 mM of L-glu-di-*tert*-butyl ester free base as substrate. The first mixture further contained 1x (=340 mM) sodium acetate, the second mixture contained 1.5x (=510 mM) sodium acetate, and the third mixture contained 2x (=680 mM) sodium acetate. The reactions were run with the pH adjusted to 5.0 and the reaction temperature was maintained at 50°C. The reactions were run for about 43 to about 65 hours. The results shown in TABLE V indicate that the conversion of the diester to γ-monoester depended on the concentration of sodium acetate. The addition of 1x concentration of sodium acetate resulted in the conversion of diester to mainly diacid and γ-monoester. The amount of diacid recovered was about twice as much as the amount of γ-monoester. The addition of 1.5x concentration of sodium acetate resulted in a higher percent conversion to γ-monoester, and the addition of 2x concentration of sodium acetate resulted in the a highest percent conversion to γ-monoester, (83.47%).

**TABLE V:**

| **Conversion of diester using different concentration of sodium acetate** | | | | | |
|---|---|---|---|---|---|
| **Sodium acetate concentration** | | **Product** | | | |
| | **diester mM** | **γ-monoester mM** | **α-monoester mM** | **diacid mM** | **% conversion to γ-monoester** |
| 1X | 1.00 | 127.09 | 1.28 | 261.67 | 37.38 |
| 1.5X | 10.33 | 265.10 | 13.24 | 65.18 | 77.97 |
| 2X | 5.45 | 283.80 | 189.43 | 108.54 | 83.47 |

### EXAMPLE 6

### Non-enzymatic hydrolysis at different pH

Four sets of reactions were initiated by mixing 30 mM of L-glu-di-*tert*-butyl ester with 100 mM of potassium phosphate (3 sets) or sodium acetate (1 set). The pH of the first 3 sets of the reaction was adjusted to 6.5, 6.0, and 5.5, while the pH of the last set was adjusted to 5.5. The reactions were run at a temperature of 40°C for about 46 hours. Samples were taken at various intervals and analyzed. The results which are presented in TABLE VI, generally indicate that under the specified reaction condition, L-glu-di-*tert*-butyl ester was converted to mainly γ-monoester and a small amount of α-monoester, and L-glu (diacid). The data further indicates that in the presence of potassium phosphate, the percent conversion of the diester to γ-monoester increased from 48.56% to 92.45% with the increased pH from pH 5.5 to 6.5. However, when sodium acetate was used in place of potassium phosphate, the percent conversion was high (93.53%) at a lower pH (5.5).

**TABLE VI:**

| **The pH effect on the conversion of the diester**. | | | | | |
|---|---|---|---|---|---|
| | | **Product** | | | |
| **pH** | **diester mM** | **γ-monoester mM** | **α-monoester mM** | **diacid mM** | **% conversion to γ-monoester** |
| 6.5^{a} | 5.99 | 92.45 | 1.28 | 37.72 | 92.45 |
| 6.0^{a} | 3.42 | 58.06 | 1.39 | 43.36 | 58.06 |
| 5.5^{a} | 2.84 | 48.56 | 1.53 | 9.38 | 48.56 |
| 5.5^{b} | 6.64 | 93.53 | 1.13 | 18.62 | 93.53 |

| | | | | | |
|---|---|---|---|---|---|
| a: in the presence of potassium phosphate | | | | | |
| b: in the presence of sodium acetate | | | | | |

While the invention has been illustrated and described in detail in the drawings and foregoing description, the same is to be considered as illustrative and not restrictive in character. It should be understood that only the exemplary embodiments have been shown and described and that all changes and modifications that come within the spirit of the invention are desired to be protected.

## Claims

1. A method for preparing an amino acid monoester without the use of an enzyme comprising:
providing an amino acid diester comprising an α-ester group and an ω-ester group; and
hydrolyzing said amino acid diester in the presence of at least on additive and under reaction conditions that affect selective hydrolysis of said α-ester group to form an amino acid ω-monoester, said reaction conditions compring a pH of between 3.0 and 6.5.

2. The method of claim 1 wherein said amino acid diester comprises at least one of a free base, and an amino acid diester salt.

3. The method of claim 2 wherein said free base includes an amino acid-di-tert-butyl ester.

4. The method of claim 3 wherein said amino acid-di-tert-butyl ester comrises aspartic acid-di-tert-butyl ester, and said amino acid ω-monoester comrises aspartic acid-β-tert-butyl ester.

5. The method of claim 3 wherein said amino acid-di-tert-butyl ester comprises glutamic acid-di-tert-butyl ester, and said amino acid w-monoester comprises glutamic acid-γ-tert-butyl ester.

6. The method of claim 3 wherein said amino acid-di-tert-butyl ester comprises a synthetic amino acid-di-tert -butyl ester

7. The method of claim 1 or 2 wherein said amino acid diester comprises: 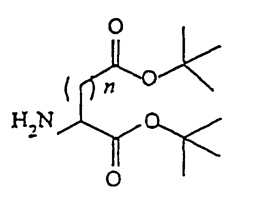 wherein n = 1-6 carbons, said each carbon forming an alkyl group.

8. The method of claim 1 or 2 wherein said amino acid diester comprises: 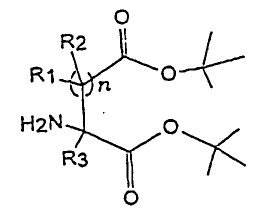 wherein n = 1-6 carbons, an each of R1, R2 and R3 comprising at least one of a hydrogen atom, an aryl group, an alkyl group, and an aralkyl group.

9. The method of claim 8 wherein said alkyl group comprises at least one of a methyl group, an ethyl group, a propyl group, a butyl group, and a pentyl group.

10. The method of claim 8 wherein said aryl group comprises at least one of a phenyl group, a 1 -naphthyl group, and a 2-naphthyl group.

11. The method of claim 8 wherein said aryl group contains at least one heteroatom.

12. The method of claim 11 wherein said heteroatom comprises at least one of a pyridyl group, a furyl group, a thienyl group, a imidazoyl group, and a pyrimidyl group.

13. The method of any one of claims 8 to 12 wherein at least one of said R1 and R2 comprises an acyl group (-COR).

14. The method of claim 13 wherein the R of said (-COR) comprises at least one of CH₃, C₂H₅, C₃H_{7,} C₄H₉, CH₂C₆H₅, and C₆H₅.

15. The method of any one of claims 8 to 12 wherein at last one of said R1 and R2 is a halogen.

16. The method of claim 15 wherein said halogen comprises at least one of fluorine (F), chlorine Cl), bromine (Br) and iodine (I).

17. The method of claim 1 wherein said amino acid diester comprises an amino acid diester salt.

18. The method of claim 17 wherein said amino acid diester salt comprises an amino acid diester hydrochloride.

19. The method of claim 18 wherein said amino acid diester hydrochloride comprises aspartic acid-di-tert-butyl ester hydrochloride.

20. The method of claim 18 wherein said amino acid diester hydrochloride comprises glutamic acid-di-tert-butyl ester hydrochloride.

21. The method of any one of claims 18 to 20 wherein said at least one additive is present at a concentration of about 2 molar equivalents of said amino acid diester hydrochloride.

22. The method of any one of claims 1 O 21 wherein said at least one additive is present at a concentration of between about 0.5 to about 2.5 molar equivalents of the amino acid diester.

23. The method of any one of claims 1 to 22 wherein said at least one additive acts as a buffer at the pH range between about 3.0 and about 6.5.

24. The method of any one of claims 1 to 23 wherein said at least one additive comprises at least one of sodium acetate, sodium citrate, sodium formate, sodium succinate, sodium carbonate, sodium bicarbonate, sodium phosphate, sodium biphosphate, and potassium phosphate.

25. The method of claim 24 wherein said at least one additive comprises sodium acetate.

26. The method of any one of claims 1 to 25 wherein said at least one additive comprises an acid additive neutralized with a base additive.

27. The method of claim 26 wherein said acid additive comprises at least one of organic acid and inorganic acid.

28. The method of claim 26 or 27 wherein said acid additive comprises at least one of acetic acid, citric acid, formic acid, succinic 'acid, carbonic acid and phosphoric acid.

29. The method of any one of claims 26 to 28 wherein said base additive comprises at least one of organic base and inorganic base.

30. The method of any one of claims 26 to 29 wherein said base additive comprises an alkali metal hydroxide.

31. The method of claim 30 wherein said alkali metal hydroxide comprises at least one of sodium hydroxide, potassium hydroxide, calcium hydroxide, and ammonium hydroxide.

32. The method of any one of claims 26 to 29 wherein said base additive comprises at least one of Tris (hydoxymethyl)aminomethane, triethylamine, and trimethyl amine.

33. The method of any one of claims 1 to 32 wherein said reaction conditions comprise a temperature of at least about 25°C.

34. The method of claim 33 wherein said reaction conditions comprise a temperature of between about 25°C and about 60°C.

35. The method of claim 34 wherein said reaction conditions comprise a temperature of about 50°C.

36. The method of any one of claims 1 to 35 wherein said reaction conditions comprise a pH about 4.9.

37. The method of any one of claims 1 to 36 wherein said reaction conditions comprise a vapor pressure of at least about 0.276 Bar (4 psi).

38. The method of claim 37 wherein said reaction conditions comprise a vapor pressure of between about 0.276 Bar (4 psi) and about 1.724 Bar (25 psi).

39. A method for preparing an amino acid monoester comprising:
providing an amino acid diester comprising an α-ester group and an ω-ester group; and
hydrolyzing said amino acid diester to form an amino acid ω-monoester in the presence of at least one non-enzyme additive and under reaction conditions comprising a tempereature of about 25°C to about 60°C.

40. The method of claim 39 wherein the at least one non-enzyme additive comprises at least one of a salt additive, an acid additive, an a base additive.

41. A method for preparing an amino acid monoester comprising:
providing an amino acid diester comprising an α-ester group and an ω-ester group; and
hydrolyzing said amino acid diester to form an amino acid ω-monoester in the presence of at least one additive and under reaction conditions comprising a temperature of about 25°C to about 60°C, a pH of about 3.0 to about 6.5, and a vapor pressure of about 0.276 Bar (4 pri) to about 1.724 Bar (25psi).

42. The amino acid ω-monoester produced by the process of any one of claims 1 - 41.
